Europäisches Patentamt

**European Patent Office**

Office européen des brevets

(11) Veröffentlichungsnummer: **0 102 558**
**B1**

(12) **EUROPÄISCHE PATENTSCHRIFT**

(45) Veröffentlichungstag der Patentschrift:
13.11.85

(21) Anmeldenummer: **83107935.5**

(22) Anmeldetag: **11.08.83**

(51) Int. Cl.⁴: **C 07 H 15/04**

(54) Verfahren zur Herstellung von Alkylglucosiden.

(30) Priorität: **03.09.82 DE 3232791**

(43) Veröffentlichungstag der Anmeldung:
**14.03.84 Patentblatt 84/11**

(45) Bekanntmachung des Hinweises auf die Patenterteilung:
**13.11.85 Patentblatt 85/46**

(84) Benannte Vertragsstaaten:
**AT BE CH DE FR GB IT LI NL SE**

(56) Entgegenhaltungen:
**FR - A - 2 017 240**
**US - A - 4 298 728**

**CHEMICAL ABSTRACTS, Band 47, 1952, Spalte 11780i,
Columbus, Ohio, USA, N.M. RUBANOVICH: "New
method of potato starch hydrolysis"**

(73) Patentinhaber: **BASF Aktiengesellschaft,
Carl-Bosch-Strasse 38, D-6700 Ludwigshafen (DE)**

(72) Erfinder: **Lorenz, Klaus, Dr., Ludwig-Uhland-Strasse 16,
D-6520 Worms 1 (DE)**
Erfinder: **Balzer, Wolf-Dieter, Dr., Bruesseler Ring 34,
D-6700 Ludwigshafen (DE)**
Erfinder: **Wolf, Helmut, Im Zollstock 6, D-6733 Hassloch
(DE)**
Erfinder: **Trieselt, Wolfgang, Dr., Alwin-Mittasch-Platz 1,
D-6700 Ludwigshafen (DE)**
Erfinder: **Busse, Gerd, Thomas-Mann-Strasse 28,
D-6704 Mutterstadt (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Niedere Alkylglucoside, vor allem, n- oder iso-Butylglucosid, stellen wichtige Zwischenprodukte zur Herstellung höherer Alkylglucoside, die bekanntlich als alkalistabile Tenside zum Einsatz gelangen, dar. Die hydrophilen und hydrophoben Anteile im Molekül verleihen den niederen Alkylglucosiden außerdem die typischen Eigenschaften von Lösungsvermittlern für hydrophobe Stoffe.

Unter dem Begriff niedere Alkylglucoside sind Gemische aus Alkylmonoglucosid und Alkyloligoglucosiden zu verstehen, wobei die Oligoglucoside in der Regel 2 bis 10 Glucoseeinheiten enthalten. Dem Fachmann ist bekannt, daß bei der üblichen Acetalisierung von Glucose in Gegenwart eines sauren Katalysators derartige Gemische entstehen.

Die bisherigen Methoden zu ihrer Herstellung bestanden generell darin, daß man Glucose mit $C_3$- bis $C_5$-Alkanolen, zumeist mit Butanolen, in Gegenwart eines sauren Katalysators acetalisierte. Aus der DE-AS 1 943 689 ist ein Verfahren zur Herstellung von Laurylglucosid bekannt, das zweistufig verläuft und bei dem man in der ersten Stufe Glucose mit Butanol in Gegenwart einer starken Säure, wie Schwefelsäure oder p-Toluolsulfonsäure acetalisiert, wobei die Butylglycoside, genau genommen ein Gemisch von Butylmonoglucosid und Butyloligoglucosiden, als Zwischenprodukte in der Reaktionslösung anfallen, aus der sie isoliert oder direkt zu höheren Alkylglycosiden umacetalisiert werden können.

Der Nachteil, der dieser Methode anhaftet, besteht darin, daß bei der Acetalisierung der Glucoside, die im allgemeinen bei der Siedetemperatur des Butanols unter Auskreisung des gebildeten Reaktionswassers abläuft, dunkelgefärbte Nebenprodukte entstehen, die später in einem zusätzlichen Arbeitsgang, z. B. durch Bleichen, entfernt werden müssen. Diese Nebenprodukte entstehen bekanntlich immer, wenn Kohlenhydrate mit Säuren behandelt werden, durch Abspaltung von Wasser im Rahmen einer Konkurrenzreaktion.

Das Ziel der Erfindung bestand darin, ein Verfahren zu entwickeln, das zu möglichst nebenproduktfreien, hellfarbigen $C_3$- bis $C_5$-Alkylglycosiden führt, die problemlos isoliert oder direkt zu höheren Alkylglycosiden weiterverarbeitet werden können.

Dieses Ziel wurde überraschenderweise mit einem Verfahren erreicht, wie es in den Patentansprüchen definiert ist.

Es besteht darin, daß man der Reaktionslösung vor oder während der Reaktion ein Alkalimetallsalz einer Borsäure in einer Menge zusetzt, die dem sauren Katalysator mindestens äquivalent ist. Dadurch wird die katalytisch wirkende Säure in ihr Alkalimetallsalz und die freiwerdende Borsäure in einen Glucose/Borsäurekomplex überführt, der nunmehr als komplexe Lewis-Säure seinerseits die Rolle des sauren Katalysators übernimmt, wobei wesentlich heller gefärbte Produkte entstehen. Dieser Befund ist insofern überraschend, als man bisher eine Acetalisierung von Glucose nur in Gegenwart von $H^{\oplus}$-Ionen in Konzentrationen erwarten konnte, die einem pH-Wert von $<3$ entsprechen.

Zweckmäßigerweise wird das Alkalimetallsalz einer Borsäure nach Auflösung der Glucose in dem Gemisch aus verwendetem Alkohol und saurem Katalysator zugesetzt.

Die hellgefärbten Produkte können problemlos durch weitere Umacetalisierung in höhere Alkylglucoside überführt werden.

Es hat sich herausgestellt, daß Alkylglucoside, die ohne den Zusatz der Borverbindungen hergestellt und erst nach der Entfernung des Reaktionswassers mit einem Bleichmittel, wie Wasserstoffperoxid oder Natriumperborat, behandelt waren, wesentlich dunkler gefärbt waren als die erfindungsgemäß erhaltenen Produkte.

Das Vefahren ist einfach durchzuführen und läuft beispielsweise in der Weise ab, daß man zunächst ein $C_3$- bis $C_5$-Alkanol, insbesondere ein Propanol, einen Amylalkohol, bevorzugt iso- oder n-Butanol, mit Glucose und dem sauren Katalysator vermischt und die Mischung unter Rückflußkühlung solange erhitzt, bis eine klare Lösung entstanden ist — im allgemeinen genügen ca. 15 bis 45 Minuten.

Der Alkohol kann gegenüber der Glucose in äquivalenten Mengen vorliegen, besser ist es jedoch ihn wegen der leichteren Auflösbarkeit der Glucose im Überschuß einzusetzen. Besonders günstig ist es, auf einem Teil Glucose 1,5 bis 5 Teile Alkohol zu verwenden. Die Glucose wird zweckmäßigerweise in der wasserfreien Form eingesetzt.

Als saure Katalysatoren setzt man die für den in Rede stehenden Zweck die üblicherweise zu verwendenden Säuren an, und es seien z. B. Schwefelsäure, Phosphorsäure, Salzsäure oder p-Toluolsulfonsäure genannt, wobei die letztere bevorzugt wird. Die sauren Katalysatoren sind in der Reaktionsmischung — bezogen auf Glucose — zu ca. 0,2 bis 5, vorzugsweise 0,5 bis 3 Gew.-% anwesend.

Dieser Reaktionsmischung setzt man, wenn sie — vorzugsweise — auf ca. 80 bis 110° C abgekühlt ist, die Alkalimetallsalze einer Borsäure zu, und zwar in mindestens der Menge, die erforderlich ist, um die sauren Katalysatoren vollständig zu neutralisieren, d. h. in äquivalenten Mengen. Je nach der Menge an anwesendem sauren Katalysator sind dies — bezogen auf Glucose — 0,5 bis 5 Gew.-%.

Unter Alkalimetallsalzen einer Borsäure versteht man im erfindungsgemäßen Sinne z. B. Borate des Kaliums oder Natriums, die zweckmäßigerweise in Form ihrer Hydrate zum Einsatz gelangen. Bevorzugt wählt man Natriummetaborattetrahydrat und Dinatriumtetraboratdekahydrat (Borax). Besonders günstige Resultate erzielt man mit solchen Boraten, die Wasserstoffperoxid gebunden enthalten (peroxohydratisierte Borate), da das während der Reaktion freiwerdende Wasserstoffperoxid noch zusätzlich bleichend wirkt, was diese Verbindung, wie oben bemerkt, nach der Reaktion und allein während der Reaktion eingesetzt nicht in befriedigendem Maße zu leisten vermag.

Als peroxyhydratisiertes Borat wird bevorzugt die unter dem (nicht ganz korrekten) Name »Natriumperborat« bekannte Verbindung eingesetzt. Nach dem Zusatz der Borate verfährt man in üblicher Weise, indem man das Wasser, vorzugsweise in Gegenwart von ca. 3 bis 5 Gew.-% — bezogen auf den Gesamtansatz — an Xylol (als Schleppmittel), destillativ abtrennt (»auskreist«), das überschüssige Alkanol abdestilliert, gegebenenfalls neutralisiert und das gebildete Glucosid isoliert oder es direkt weiterverarbeitet.

Die erhaltenen Glucoside fallen — besonders wenn sie in Gegenwart von peroxohydratisierten Boraten erhalten worden sind — nahezu farblos an und können daher direkt verwendet oder mit höheren Alkoholen zu höheren Alkylglycosiden weiter umgesetzt werden. Die Glycoside, insbesondere das n-Butylglycosid, können als Lösungsvermittler, vor allem wegen ihrer Reinheit im kosmetischen Bereich dienen, außerdem sind sie hervorragende Ausgangsprodukte zur Herstellung höherer Alkylglycoside, die man ebenfalls in wesentlich mehr aufgehelltem Zustand nach allen bekannten Methoden erhalten kann.

Die nun folgenden Beispiele erläutern die Erfindung. Teile sind Gewichtsteile.

## Beispiel 1

810 Teile n-Butanol, 568 Teile wasserfreie Glucose und 9 Teile p-Toluolsulfonsäure wurden $^1/_2$ Stunde unter Rückfluß erhitzt, wobei die Glucose in Lösung ging. Anschließend wurde auf 100°C abgekühlt. Dem Ansatz wurden sodann 7,4 Teile Natriumperborattetrahydrat zugefügt, wobei sich die Reaktionslösung aufhellte. Anschließend fügte man 60 Teile Xylol zu und kreiste innerhalb von 2 Stunden das Reaktionswasser (mit Xylol als Schlepper) aus. Nach der destillativen Abtrennung des überschüssigen n-Butanols blieb ein nahezu farbloses Produkt zurück. Jodfarbzahl: 1 (nach DIN 6162).

## Beispiel 2 (Vergleichsbeispiel)

Es wurde gemäß Beispiel 1 verfahren, das Perborat jedoch weggelassen. Nach Auskreisen des Reaktionswassers wurde ein braunes Öl erhalten, welches nach Zusatz von 7,4 Teilen Natriumperborattetrahydrat oder einer äquivalenten Menge $H_2O_2$ und destillativer Abtrennung des überschüssigen n-Butanols nur bis mittelgelb aufgehellt werden konnte. Jodfarbzahl: 10 bis 15 (DIN 6162).

## Beispiel 3

Es wurde gemäß Beispiel 1 verfahren, anstelle von Na-perborat jedoch 6,6 Teile Na-metaborattetrahydrat zugegeben. Jodfarbzahl: 5 bis 7 (DIN 6162).

## Beispiel 4

Es wurde gemäß Beispiel 1 verfahren, anstelle von Na-perborat jedoch 9,2 Teile Borax (Dinatriumtetraboratdekahydrat) zugegeben. Jodfarbzahl: 5 bis 7 (DIN 6162).

## Patentansprüche

1. Verfahren zur Herstellung von Alkylglucosiden durch Acetalisierung von Glucose mit der zumindest äquivalenten Menge an einem $C_3$- bis $C_5$-Alkanol in Gegenwart eines sauren Katalysators, dadurch gekennzeichnet, daß man die Acetalisierung in Gegenwart einer dem sauren Katalysator mindestens äquivalenten Menge eines Alkalimetallsalzes einer Borsäure durchführt.

2. Verfahren nach Anspruch 1, dadurch gekennzeichnet, daß man die Acetalisierung in Gegenwart eines hydratisierten und/oder peroxohydratisierten Alkalimetallsalzes einer Borsäure durchführt.

3. Verfahren nach Anspruch 1 oder 2, dadurch gekennzeichnet, daß man die Acetalisierung in Gegenwart von Natriummetaborat, Borax und/oder Natriumperborat durchführt.

4. Verfahren nach Ansprüchen 1 bis 3, dadurch gekennzeichnet, daß man das Alkalimetallsalz der nach Erhitzen von Alkanol, Glucose und saurem Katalysator erhaltenen Lösung zufügt und anschließend die Reaktion in an sich bekannter Weise unter destillativer Abtrennung des gebildeten Reaktionswassers zu Ende führt.

## Claims

1. A process for the preparation of an alkylglucoside by acetalization of glucose with an equivalent amount or an excess of a $C_3—C_5$-alkanol in the presence of an acidic catalyst, wherein the acetalization is carried out in the presence of an alkali metal salt of a boric acid used in an amount which is quivalent to or in excess of the amount of the acidic catalyst.

2. A process as claimed in claim 1, wherein the acetalization is carried out in the presence of a hydrated and/or peroxohydrated alkali metal salt of a boric acid.

3. A process as claimed in claim 1 or 2, wherein the acetalization is carried out in the presence of sodium metaborate, borax and/or sodium perborate.

4. A process as claimed in claims 1 to 3, where in the alkali metal salt is added to the solution obtained after the alkanol, glucose and acidic catalyst have been heated, and the reaction is then completed in a conventional manner, the water of reaction formed being separated off by distillation.

## Revendications

1. Procédé de préparation d'alkyl-glycosides par acétalisation du glucose avec une proportion au moins équivalente d'un alcanol en C$_3$ à C$_5$ en présence d'un catalyseur acide, caractérisé en ce que l'acétalisation est effectuée en présence d'une proportion au moins équivalente au catalyseur acide d'un sel d'un métal alcalin d'un acide borique.

2. Procédé suivant la revendication 1, caractérisé en ce que l'acétalisation est réalisée en présence d'un sel hydraté et (ou) peroxo-hydraté d'un métal alcalin d'un acide borique.

3. Procédé suivant la revendication 1 ou 2, caractérisé en ce que l'acétalisation est réalisée en présence de méta-borate de sodium, de borax et (ou) de perborate de sodium.

4. Procédé suivant les revendications 1 à 3, caractérisé en ce que le sel de métal alcalin est ajouté à la solution obtenue par chauffage du glucose, de l'alcanol et du catalyseur acide, la réaction étant ensuite emplétée de manière connue en soi avec élimination de l'eau réactionnelle formée par distillation.